# EUROPÄISCHE PATENTSCHRIFT

(11) **EP 3 450 536 B1**
(45) Veröffentlichungstag und Bekanntmachung des Hinweises auf die Patenterteilung: **08.04.2020**
(21) Anmeldenummer: 17188374.7
(22) Anmeldetag: 29.08.2017
(51) Int. Cl.: C12M 1/107, C12M 1/00, C12M 1/06, C12M 1/34, C12M 1/36

(54) **VERFAHREN ZUR OPTIMIERUNG DES BETRIEBS EINES PFROPFENSTROMFERMENTERS ZUR ANAEROBEN VERGÄRUNG ORGANISCHER ABFÄLLE**
METHOD FOR OPTIMISING THE OPERATION OF A GRAFTING CURRENT FERMENTER FOR THE ANAEROBIC FERMENTATION OF ORGANIC WASTE
PROCÉDÉ D'OPTIMISATION DE FONCTIONNEMENT D'UN FERMENTEUR À ÉCOULEMENT PISTON POUR FERMENTATION ANAÉROBIE DE DÉCHETS ORGANIQUES

(43) Veröffentlichungstag der Anmeldung: 06.03.2019
(73) Patentinhaber: Hitachi Zosen Inova AG, 8005 Zürich (CH)
(72) Erfinder: SCHATZ, Adrian, 3172 Niederwangen (CH); EUGSTER, Marc, 8108 Dällikon (CH)
(74) Vertreter: Schaad, Balass, Menzl & Partner AG

(56) Entgegenhaltungen:
- WO-A1-2005/113469
- WO-A1-2006/079228
- WO-A1-2011/121024
- WO-A1-2016/071454
- DE-A1-102005 041 798
- DE-A1-102014 116 239

## Beschreibung

Die Erfindung betrifft ein Verfahren zur Optimierung des Betriebs eines Pfropfenstromfermenters zur anaeroben Vergärung organischer Abfälle sowie einen entsprechend ausgebildeten Pfropfenstromfermenter.

Verfahren zur anaeroben Vergärung organischer Abfälle sind dem Fachmann bekannt und haben gerade in den letzten Jahren an Bedeutung zugenommen, da sie einerseits eine ökologische Abfallbewirtschaftung erlauben, andererseits aber auch aus ökonomischer Sicht interessant sind, weil sich damit bedeutende Mengen an Biogas erzeugen lassen.

Unter den bekannten anaeroben Vergärungsverfahren können grundsätzlich Flüssig- oder Nass-Vergärungsverfahren von den sogenannten Trocken-Vergärungsverfahren unterschieden werden. Ein Nass-Vergärungsverfahren wird etwa in WO 2011/121024 beschrieben. Dabei wird einem Behälter Substrat mittels eines Aufgabesystems zugeführt, wobei im Behälter mindestens zwei Rührwerke angeordnet sind, deren Propeller über Antriebe in Rotation versetzt werden. Zudem beschreibt WO 2016/071454 eine Rühreinrichtung für einen Fermenter einer Biogasanlage, welche ein Gehäuse und eine Antriebseinrichtung zum drehbaren Antreiben wenigstens eines Rührflügels der Rühreinrichtung umfasst. Während Nass-Vergärungsverfahren üblicherweise in einem oder mehreren vertikal ausgerichteten Fermentern durchgeführt werden, wird bei den sogenannten Trocken-Vergärungsverfahren das Gärgut in der Regel in einem horizontal ausgerichteten Fermenter vergoren.

Der Trockensubstanzanteil des Gärguts liegt dabei wesentlich höher als bei Nass-Vergärungsverfahren; nichtsdestotrotz weist das Gärgut auch bei der TrockenVergärung einen namhaften Flüssigkeitsanteil auf.

Trocken-Vergärungsverfahren können insbesondere in einem Pfropfenstromfermenter durchgeführt werden, wie sie etwa aus der EP-A-0476217 bekannt sind. Solche Fermenter umfassen in der Regel einen langgestreckten, liegenden Fermentertank mit einem an einem Ende vorgesehenen Fermentereinlass und einem am gegenüberliegenden Ende angeordneten Fermenterauslass.

Die zu vergärenden organischen Abfälle werden einlassseitig zerkleinert eingegeben und mit bereits vergorenem Gut und/oder Presswasser aus der Gärrest-Aufbereitung geimpft. Hierdurch wird das zu vergärende Gut mit Methanbakterien angereichert. Im Fermentertank werden die Abfälle nun bei kontrollierter Durchmischung unter Bildung von Biogas abgebaut und anschliessend, d.h. nach dem Verlassen durch den Fermenterauslass, einer Gärrest-Aufbereitung und weiter einer aeroben Verrottung zugeführt.

Das Rührwerk solcher Pfropfenstromfermenter muss einerseits gewährleisten, dass das Gärgut im Sinne einer möglichst optimalen Vergärung gut mit Methanbakterien durchmischt wird. Zudem soll durch die Durchmischung bzw. die sich ergebende stetige Oberflächenerneuerung eine gute Entgasung gewährleistet werden.

Weiter muss durch das Rührwerk sichergestellt werden, dass schwerere Feststoffbestandteile des Gärguts, die am Boden des Fermentertanks sedimentieren können, wieder in die oberen Schichten transportiert werden können und somit auch diese Bestandteile des Gärguts durch den Fermentertank hindurchtransportiert werden und diesen zu gegebener Zeit verlassen.

Mit dem Ziel, die Verweilzeit des Gärguts im Fermenter zu verringern und damit den möglichen Durchsatz zu erhöhen, befasst sich etwa die WO 2005/113469, gemäss welcher Frischgut oder Gärgut über mehrere Eintrittsöffnungen zugeführt und/oder Gärgut über mehrere Gärgut-Austragsöffnungen abgezogen werden kann.

Gerade bei Pfropfenstromfermentern stellt sich häufig das Problem, dass sich an der Rührwelle Anteile des Gärguts verfestigen können und an dieser haften bleiben. Die damit einhergehende Gewichtszunahme führt automatisch zu einer höheren Belastung der Rührwelle und damit zu deren Durchbiegen. Dies wiederum kann dazu führen, dass die an der Rührwelle angeordneten Paddel bzw. am radial äusseren Ende der Paddel angeordneten Schaufeln an der Wand des Fermentertanks reiben, wodurch das Drehmoment an der Welle nochmals steigt und zudem Abnutzungen an der Fermentertankwand auftreten. Desweiteren können trotz vorgängiger Zerkleinerung der biogenen Abfälle immer wieder längliche Feststoffe wie Seile, Drähte oder schlingenartige Grüngutabfälle in den Fermenter gelangen und sich während des Betriebs um die Rührwelle herumwickeln. Diese Effekte können zu einer Beeinträchtigung des Fermenterbetriebs und im schlimmsten Fall zu dessen Ausfall führen.

Ausgehend von den oben beschriebenen Problemen schlägt die EP-A-1841853 ein Verfahren zum Betrieb eines pfropfstrombetriebenen Fermenters vor, bei dem a) das Rührwerk in einer Richtung gedreht wird, wobei mit V-förmig angeordneten Scharblechen eine pflügende Wirkung erzielt wird, b) das Rührwerk in einer gegenläufigen Richtung gedreht wird, wobei die Schaufeln eine fördernde Wirkung erzielen, und c) das Rührwerk still steht.

Allerdings trägt das in EP-A-1841853 beschriebene Verfahren der Inhomogenität des Gärguts nur beschränkt Rechnung. Dies kann dazu führen, dass aufgrund einer ungenügenden Durchmischung oder einer zu kurzen Verweilzeit das Gärgut im Fermentertank nicht optimal vergoren und entgast wird. Auf der anderen Seite ist der Energieaufwand des Rührwerks für eine gute Durchmischung und Förderung höher als nötig, was sich in einer suboptimalen Energiebilanz niederschlägt.

Vor diesem Hintergrund stellt sich erfindungsgemäss die Aufgabe, ein Verfahren zur Optimierung des Betriebs eines Pfropfenstromfermenters zur anaeroben Vergärung organischer Abfälle zu Verfügung zu stellen. Insbesondere soll eine optimale Vergärung und Entgasung bei möglichst geringem Energieaufwand gewährleistet werden.

Die Aufgabe wird durch das erfindungsgemässe Verfahren gemäss Anspruch 1 gelöst. Bevorzugte Ausführungsformen der Erfindung sind in den abhängigen Ansprüchen definiert.

Gemäss Anspruch 1 umfasst der Pfropfenstromfermenter, dessen Betrieb es zu optimieren gilt, einen horizontal ausgerichteten Fermentertank und ein Rührwerk. Das Rührwerk seinerseits umfasst eine das Innere des Fermentertanks axial durchquerende Rührwelle und mehrere an der Rührwelle angeordnete, radial abstehende Paddel sowie einen Antrieb. Mittels des Rührwerks wird das Gärgut im Fermentertank bewegt. Dadurch wird das Gärgut einerseits durchmischt, andererseits wird durch die Bewegung in der Regel auch die Förderung des Gärguts vom Fermentereinlass in Richtung zum Fermenterauslass hin unterstützt.

Die angestrebte Optimierung des Betriebs wird erfindungsgemäss nun dadurch erhalten, dass
a) mindestens ein für den jeweiligen Betriebszustand des Pfropfenstromfermenters charakteristischer Parameter gemessen wird,
b) der jeweilige in a) erhaltene Messwert Aᵢₛₜ mit einem vorgegebenen Sollwert Aₛₒₗₗ verglichen wird, und
c) in Abhängigkeit von der Abweichung des Messwerts Aᵢₛₜ vom Sollwert Aₛₒₗₗ die Drehzahl der Rührwelle, die Drehrichtung der Rührwelle und/oder der Trockensubstanzanteil des Gärguts eingestellt wird.

Erfindungsgemäss kann somit über die in Schritt a) und b) erfolgende Bestimmung des jeweiligen Parameters und über den Vergleich mit einem entsprechenden Sollwert überprüft werden, ob bei der vorliegenden Drehzahl und Drehrichtung der Rührwelle eine "Überforderung" oder eine "Unterforderung" des Systems vorliegt. Liegt eine solche vor, kann dieser über Einstellung der Drehzahl bzw. der Drehrichtung gezielt entgegengewirkt werden. Alternativ oder zusätzlich dazu kann auch der Trockensubstanzanteil des Gärguts eingestellt werden, um dessen Durchsatz zu optimieren. Dies erlaubt letztendlich eine optimale Behandlung des Gärguts, wobei der hierzu aufgewendete Energieaufwand gleichzeitig minimal gehalten wird.

Das Verfahren bzw. der Propfenstromfermenter der vorliegenden Erfindung ist auf die Trockenvergärung ausgelegt. Somit unterscheidet sich die Erfindung grundlegend von den eingangs beschriebenen Nass-Vergärungsverfahren bzw. den hierfür vorgesehenen vertikalen Anlagen, für die beispielshalber auf die DE 10 2014 116 239 A1 verwiesen wird.

Wie erwähnt kann in Schritt c) des erfindungsgemässen Verfahrens der Trockensubstanzanteil des Gärguts eingestellt und damit letztendlich dessen Förderbarkeit angepasst werden. In der Regel erfolgt dies über die Einstellung der Menge an in den Fermentertank eingeführtem und somit dem Gärgut zugegebenen Befeuchtungsmittel. Denkbar ist aber auch, den Trockensubstanzanteil über die Menge des in den Fermentertank eingeführten Gärguts einzustellen.

Konkret liegt der Trockensubstanzanteil des Gärguts vorzugsweise im Bereich zwischen 5 bis 99%, bevorzugter zwischen 15 bis 40 %, und somit deutlich über dem Trockensubstanzanteil, der in Nassvergärungsanlagen vorliegt.

Im Kontext der vorliegenden Erfindung wird der Begriff "Abfall" für das bei der Einspeisung vorliegende, zu vergärende Material verwendet, während als "Gärgut" das im Fermentertank vorliegende, angeimpfte und vergärende Material auf Basis der Abfälle bezeichnet wird.

Bei den Abfällen bzw. dem Gärgut im Sinne der vorliegenden Erfindung handelt es sich insbesondere um ein relativ inhomogenes Material, womit gemeint ist, dass sich der Trockensubstanzanteil aus Feststoffpartikeln bzw. Feststoffbestandteilen unterschiedlicher Grösse und Form zusammensetzt. Insbesondere können relativ grossvolumige Feststoffbestandteile darin enthalten sein. Typischerweise handelt es sich bei den organischen Abfällen um Mischungen aus Abfällen wie Haus-, Garten-, Landwirtschafts-, Industrie- und Grüngutabfälle, Speisereste und tierische Exkremente wie Festmist. Die erfindungsgemäss erhaltenen Vorteile schlagen gerade bei solch inhomogenen Materialien besonders zu Buche, da die für das System optimale Drehzahl und Drehrichtung der Rührwelle sowie der optimale Trockensubstanzanteil permanenten Schwankungen unterliegen können, welche erfindungsgemäss ausgeglichen werden können.

Der Begriff "Sollwert", wie er im Kontext der vorliegenden Erfindung verwendet wird, schliesst insbesondere einen Sollwertbereich mit ein. Mit anderen Worten liegt erfindungsgemäss dann eine Abweichung des Messwerts Aᵢₛₜ vom Sollwert Aₛₒₗₗ vor, wenn der Messwert höher als die Obergrenze des Sollwertbereichs bzw. tiefer als die Untergrenze des Sollwertbereichs liegt.

Im Besonderen wird erfindungsgemäss keine Soll-Lastkurve hinterlegt, wie dies etwa gemäss der DE 10 2014 116 239 A1 gelehrt wird. Das Hinterlegen einer Soll-Lastkurve würde im Verfahren der vorliegenden Erfindung gerade auch in Anbetracht der Inhomogenität des Gärguts technisch nur beschränkt Sinn machen.

Wie erwähnt wird in Schritt c) die Drehzahl der Rührwelle, die Drehrichtung der Rührwelle und/oder der Trockensubstanzanteil des Gärguts in Abhängigkeit von der Abweichung des Messwerts Aᵢₛₜ vom Sollwert Aₛₒₗₗ eingestellt. Gemäss einer bevorzugten Ausführungsform wird die Drehzahl und/oder die Drehrichtung der Rührwelle bzw. der Trockensubstanzanteil des Gärguts dabei nicht nur einmalig eingestellt, sondern auch geregelt. Mit anderen Worten wird die Auswirkung der Änderung der Drehzahl und/oder der Drehrichtung bzw. des Trockensubstanzanteils auf die Abweichung des Messwerts Aᵢₛₜ vom Sollwert Aₛₒₗₗ fortlaufend überwacht und ausgehend davon die Drehzahl, die Drehrichtung und/oder der Trockensubstanzanteil weiter angepasst oder belassen, sodass ein geschlossener Wirkungsablauf vorliegt.

Vorzugsweise liegt der Bereich, innerhalb welchem die Drehzahl der Rührwelle eingestellt wird, zwischen 0 und 10, vorzugsweise zwischen 0 und 1, besonders bevorzugt zwischen 0,2 bis 0,6 Umdrehungen pro Minute. Die Drehzahl der Rührwelle unterscheidet sich somit ganz wesentlich von der Drehzahl eines Propellers, wie er etwa in einem Nassvergärungsverfahren zur Anwendung kommen kann und wie er etwa gemäss der in der DE 10 2014 116 239 A1 beschriebenen Technologie vorliegt.

Die an der Rührwelle angeordneten Paddel bzw. die wahlweise am radial äusseren Ende der Paddel angeordneten Schaufeln dienen primär dazu, das Gärgut für eine optimale Vergärung zu durchmischen. Dabei ist denkbar, die Paddel und insbesondere die daran angeordneten Schaufeln symmetrisch auszugestalten, sodass dem Gärgut von den Paddeln bzw. Schaufeln keine aktive Förderkomponente zum Fermenterauslass hin verliehen wird. Dabei ist insbesondere denkbar, die Schaufeln hinsichtlich der Drehrichtung asymmetrisch auszugestalten, sodass sich bei einer ersten Drehrichtung eine radiale Förderwirkung auf das Gärgut ergibt und bei einer zweiten Drehrichtung eine Pflugwirkung. Bei dieser Ausführungsform entsteht der Vortrieb des Gärguts primär durch den Nachschub an Gärgut durch den Fermentereinlass, wobei die Förderung durch die mittels der Rührwelle erzeugte Bewegung des Gärguts unterstützt wird. Denkbar ist aber auch, die Schaufeln derart auszugestalten, dass dem Gärgut eine aktive, axiale Förderkomponente in Richtung Fermenterauslass verliehen wird.

Als für den Betriebszustand charakteristischer Parameter wird erfindungsgemäss A1: das Drehmoment und/oder die Leistung des Antriebs gewählt, des Weiteren wahlweise mindestens einer der folgenden Parameter A2) bis A4):
A2: mindestens eine im Fermentertank vorliegende Temperatur;
A3: der Durchfluss und/oder die Zusammensetzung des Gärguts an mindestens einem Punkt im Fermentertank; und/oder
A4: die Zusammensetzung und/oder die Menge des durch die anaerobe Vergärung erzeugten Gases.

Dabei wird das Drehmoment und/oder die Leistung des Antriebs, d.h. Parameter A1 bestimmt. Denkbar ist diesbezüglich etwa, dass die Leistung ansteuerungsseitig des Antriebs über das Produkt des Stroms und der Spannung ermittelt wird. Weiter ist denkbar, dass das Drehmoment aus der Leistung des Antriebs abgeleitet wird. Gemäss einer besonders bevorzugten Ausführungsform liegt der Antrieb in Form eines Motors, konkret eines Asynchronmotors vor, dem ein Frequenzumwandler zugeordnet ist; in diesem Fall ist bevorzugt, dass das Drehmoment in Prozent am Frequenzumwandler ermittelt wird.

Ausgehend von dem in Schritt a) erhaltenen Messwert A1ᵢₛₜ des Drehmoments und/oder der Leistung des Antriebs wird gemäss einer weiter bevorzugten Ausführungsform des erfindungsgemässen Verfahrens für den Fall, dass der in Schritt a) erhaltene Messwert A1ᵢₛₜ des Drehmoments bzw. der Leistung höher liegt als ein vorgegebener Maximalwert A1ₘₐₓ, die Drehzahl reduziert.

Dabei wird anschliessend das Drehmoment bzw. die Leistung bei reduzierter Drehzahl gemessen.

Schliesslich wird für den Fall, dass der bei reduzierter Drehzahl gemessene Wert A1_{ist,red} immer noch höher liegt als A1ₘₐₓ,
- das Drehmoment bzw. die Leistung nochmals reduziert, worauf wahlweise die Messung des Drehmoments bzw. der Leistung bei nochmalig reduzierter Drehzahl und die daraufhin vorgenommene nochmalige Reduktion der Drehzahl mindestens einmal, vorzugsweise mehrmals wiederholt werden, und/oder
- die Drehrichtung der Rührwelle geändert.

Somit wird für den Fall, dass das System überfordert ist, dieses durch Reduktion der Drehzahl und/oder durch Änderung bzw. Umkehr der Drehrichtung entlastet, wobei als Indikator für eine Systemüberforderung der Umstand herangezogen wird, dass das Drehmoment bzw. die Leistung des Antriebs einen vorgegebenen Sollwert überschreitet.

Vorzugsweise wird die Drehzahl im jeweiligen Schritt nur in geringem Masse reduziert, sodass die sich aufgrund der Drehzahlreduktion ergebende Differenz in einem Bereich von 0,001 bis 0,1 Umdrehungen liegt, vorzugweise bei ca. 0,05 Umdrehungen pro Minute. Dadurch kann gewährleistet werden, dass auch bei reduzierter Drehzahl diese nach wie vor hoch genug ist, um eine ausreichende Durchmischung des Gärguts zu gewährleisten.

Wird die Drehrichtung der Rührwelle geändert, so kann bevorzugt sein, dass nachfolgend auf diese Änderung nach einer Zeitdauer t die Drehrichtung der Rührwelle wieder geändert wird, wodurch wieder die ursprüngliche Drehrichtung erhalten wird. Dies ist insbesondere dann der Fall, wenn die Paddel und/oder unter Umständen daran angeordnete Schaufeln hinsichtlich der Drehrichtung asymmetrisch angeordnet sind, sodass sich bei einer ersten Drehrichtung eine radiale Förderwirkung auf das Gärgut ergibt und bei einer zweiten Drehrichtung eine Pflugwirkung, mittels welcher eine Auflockerung von sich stauendem Gärgut bewirkt werden kann, worauf das Gärgut - nach erneuter Drehrichtungsumkehr - wieder einfacher gefördert werden kann.

Alternativ zum oben beschriebenen Verfahren, bei dem eine Systemüberforderung festgestellt wird, ist auch denkbar, dass der in Schritt a) erhaltene Messwert A1ᵢₛₜ des Drehmoments bzw. der Leistung tiefer liegt als ein vorgegebener Minimalwert A1ₘᵢₙ, was auf eine "Unterforderung" des Systems hindeutet. In diesem Fall wird die Drehzahl der Rührwelle vorzugweise reduziert und das System somit in einen Energiesparbetrieb umgestellt, bei dem nichtsdestotrotz gewährleistet werden kann, dass die Durchmischung und die Förderung des Gärguts ausreichend hoch ist.

Die reduzierte Drehzahl bzw. der Energiesparbetrieb wird beibehalten, solange der Messwert A1ᵢₛₜ tiefer liegt als A1ₘᵢₙ. Wird festgestellt, dass der Messwert A1ᵢₛₜ den Minimalwert A1ₘᵢₙ überschreitet, wird die Drehzahl wieder angehoben, sodass A1ᵢₛₜ innerhalb des durch die Grenzwerte A1ₘᵢₙ und A1ₘₐₓ definierten Bereichs zu liegen kommt.

Nebst dem oben beschrieben Verfahren betrifft die vorliegende Erfindung gemäss einem weiteren Aspekt auch einen für das Verfahren ausgestatteten Pfropenstromfermenter gemäß Anspruch 9.

Somit umfasst der Pfropfenstromfermenter nebst dem oben beschriebenen Fermentertank und dem Rührwerk zudem mindestens eine Messeinrichtung zur Bestimmung mindestens eines für den jeweiligen Betriebszustand des Pfropfenstromfermenters charakteristischen Parameters sowie Einstellmittel, die derart ausgestaltet sind, ausgehend vom erhaltenen Messwert bzw. der Abweichung des Drehmoments und/oder der Leistung des Antriebs von einem vorgegebenen Sollwert die Drehzahl und/oder die Drehrichtung der Rührwelle einzustellen.

Vorzugsweise liegen die Einstellmittel in Form oder als Teil einer Regelungseinheit vor, die derart ausgestaltet ist, die Drehzahl und/oder die Drehrichtung der Rührwelle ausgehend vom erhaltenen Messwert bzw. der Abweichung des Messwerts vom Sollwert zu regeln.

Weiter ist bevorzugt, dass es sich beim Antrieb um einen Motor, insbesondere einen Asynchronmotor, handelt, dem ein Frequenzumwandler zugeordnet ist.

Die Erfindung wird anhand der Figuren weiter veranschaulicht.

Es zeigt:
- Fig. 1: ein Schema einer Regelung gemäss dem Verfahren der vorliegenden Erfindung; und
- Fig. 2: ein Diagramm, in welchem Drehmoment-Werte (in % am Frequenzumwandler) bei jeweils vorliegender Leistung des Antriebs eingetragen sind, und in welchem zudem Sollwerte bei unterschiedlichen Drehzahlen der Rührwelle definiert sind.

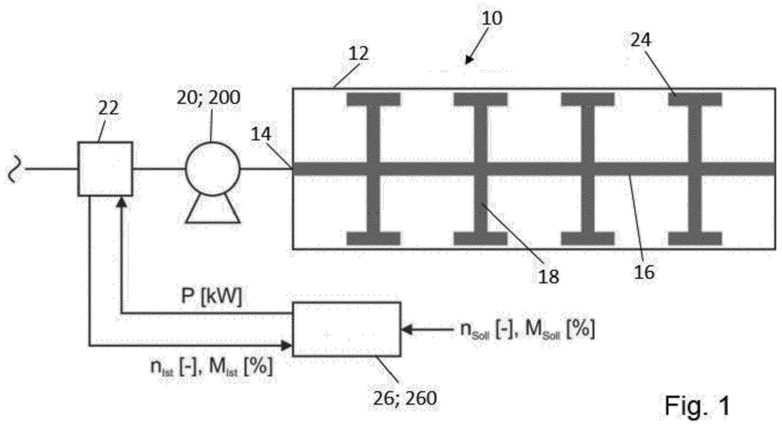

Gemäss Fig. 1 wird zur Durchführung des erfindungsgemässen Verfahrens ein Pfropfenstromfermenter 10 verwendet, der einen horizontal ausgerichteten Fermentertank 12 und ein Rührwerk 14 umfasst.

Das Rührwerk 14 umfasst eine das Innere des Fermentertanks 12 axial durchquerende Rührwelle 16 und mehrere an der Rührwelle angeordnete, radial abstehende Paddel 18 sowie einen Antrieb 20, der im konkreten Fall in Form eines Asynchronmotors 200 vorliegt, dem ein Frequenzumwandler 22 zugeordnet ist. Am radial äusseren Ende der Paddel 18 sind im Übrigen Schaufeln 24 angeordnet, welche derart ausgestaltet sind, das Gärgut zu durchmischen und dadurch die Förderung des Gärguts zum Fermenterauslass hin zu unterstützen.

In dem in Fig. 1 gezeigten Ausführungsbeispiel wird in einem Schritt a) bei vorliegender Drehzahl nᵢₛₜ[U/min] der Rührwelle 16 das Drehmoment Mᵢₛₜ[%] am Frequenzumwandler 22 gemessenen.

Der erhaltene Messwert wird in einem Schritt b) mit einem Sollwert Mₛₒₗₗ[%], konkret einem Sollwertbereich verglichen.

Resultiert aus diesem Vergleich, dass der bestimmte Wert des Drehmoments bzw. der Leistung über einem Maximalwert des Sollwertbereichs liegt (Fall A), was etwa bei einem relativ hohen Trockensubstanzgehalt des Gärguts der Fall sein kann, wird das System entlastet, indem über Einstellmittel 26 in Form einer Regelungseinheit 260 dem Frequenzumwandler 22 das Signal erteilt wird, die Drehzahl in Schritt c) auf den Wert n_{soll,neu} zu vermindern.

In der Folge wird das für die reduzierte Drehzahl n_{soll,neu} der Rührwelle 16 aufzuwendende Drehmoment bestimmt und wieder mit dem Maximalwert Mₘₐₓ verglichen.

Liegt das Drehmoment immer noch zu hoch, so deutet dies auf einen Gärgutstau hin. Diesem kann dadurch begegnet werden, dass die Reduktion der Drehzahl mit der darauffolgenden Messung des bei reduzierter Drehzahl aufzuwendenden Drehmoments und dem Vergleich des Drehmoments mit dem Maximalwert mehrmals wiederholt wird, bis das Drehmoment unter dem Maximalwert liegt. Alternativ dazu kann auch die Drehrichtung der Rührwelle geändert werden. Je nach Ausbildung der Paddel bzw. allfällig daran angeordneter Schaufeln kann somit eine pflügende Wirkung erhalten werden, um den Gärgutstau aufzulösen. Zudem kann mit Änderung der Drehrichtung, wenn sich das zu hohe Drehmoment unter anderem aus an den Paddeln angestauten Gärgutbestandteilen ergibt, eine Lockerung und letztendlich eine Entfernung dieser Bestandteile von den Paddeln erhalten werden.

Wird hingegen in Schritt b) festgestellt, dass das Drehmoment unter einem Minimalwert des Sollwertbereichs liegt (Fall B), so deutet dies auf eine "Unterforderung" des Systems hin. Auf diese wird derart reagiert, dass die Drehzahl auf eine minimale Drehzahl minimiert oder ganz abgeschaltet wird und somit der Antrieb in einen Energiesparmodus umgeschaltet wird.

Alternativ oder zusätzlich ist denkbar, aufgrund etwa der Zusammensetzung des Gärguts an mindestens einem Punkt im Fermentertank und/oder nach dem Austritt aus dem Fermentertank, Rückschlüsse zu ziehen, ob die Durchmischung im Fermentertank (bei reduzierter Drehzahl) ausreichend ist.

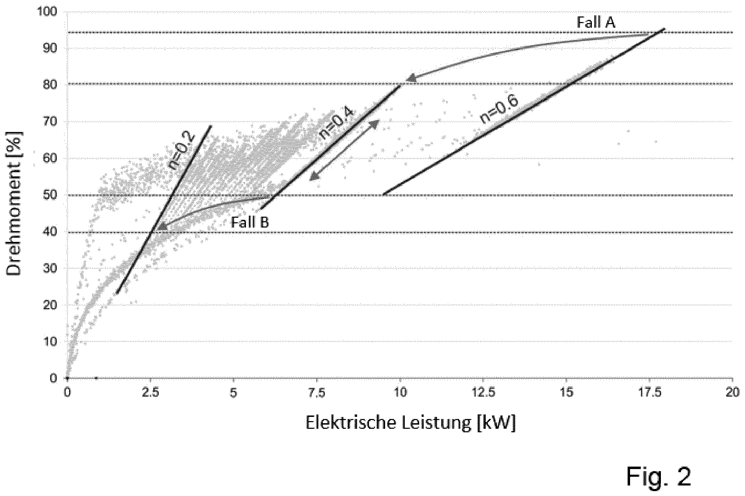

Die unterschiedlichen Szenarien sind im Diagramm gemäss Fig. 2 wiedergegeben, wobei für das Drehmoment am Frequenzumwandler ein Sollwertbereich zwischen 50 und 80% und eine minimale Drehzahl von 0,2 Umdrehungen pro Minute (U/min) vorgegeben wurden.

Ergibt die Bestimmung des Drehmoments, dass dieses bei einer Drehzahl der Rührwelle von 0,6 U/min bei 95% und somit zu hoch liegt, wird die Drehzahl vermindert, wobei im konkreten Beispiel die Drehzahl auf 0,4 U/min reduziert wird, worauf das Drehmoment erneut bestimmt und mit dem Sollwert bzw. dem Maximalwert des Sollwertbereichs verglichen wird.

Wird hingegen bei vorliegender Drehzahl von 0,4 U/min eine Leistung des Antriebs ermittelt, die unter einem vorgegebenen Minimalwert liegt, im konkreten Fall unter 6 kW, so wird die Drehzahl minimiert und das System somit in einen Energiesparbetrieb umgestellt.

In dem im Diagramm gezeigten Beispiel wird die Drehzahl sukzessive reduziert, von den erwähnten 0,4 U/min auf 0,2 U/min.

### Bezugszeichenliste

- 10: Pfropfenstromfermenter
- 12: Fermentertank
- 14: Rührwerk
- 16: Rührwelle
- 18: Paddel
- 20; 200: Antrieb; Asynchronmotor
- 22: Frequenzumwandler
- 24: Schaufeln
- 26; 260: Einstellmittel; Regelungseinheit

## Patentansprüche

1. Verfahren zur Optimierung des Betriebs eines Pfropfenstromfermenters (10) zur anaeroben Vergärung organischer Abfälle, wobei der Pfropfenstromfermenter einen horizontal ausgerichteten Fermentertank (12) und ein Rührwerk (14) umfasst, welches eine das Innere des Fermentertanks (12) axial durchquerende Rührwelle (16) und mehrere an der Rührwelle angeordnete, radial abstehende Paddel (18) sowie einen Antrieb (20; 200) umfasst, und das Gärgut mittels des Rührwerks (14) im Fermentertank (12) bewegt wird, **dadurch gekennzeichnet, dass**
a) das Drehmoment und/oder die Leistung des Antriebs (20; 200) gemessen wird,
b) der jeweilige in a) erhaltene Messwert A1ᵢₛₜ mit einem vorgegebenen Sollwert A1ₛₒₗₗ verglichen wird, und
c) in Abhängigkeit von der Abweichung des Messwerts A1ᵢₛₜ vom Sollwert A1ₛₒₗₗ die Drehzahl der Rührwelle (16), die Drehrichtung der Rührwelle (16) und/oder der Trockensubstanzanteil des Gärguts eingestellt wird.

2. Verfahren nach Anspruch 1, **dadurch gekennzeichnet, dass** die Einstellung des Trockensubstanzanteils des Gärguts über die Menge an in den Fermentertank (12) eingeführtem Befeuchtungsmittel erfolgt.

3. Verfahren nach Anspruch 1 oder 2, **dadurch gekennzeichnet, dass** in Schritt c) die Drehzahl der Rührwelle (16), die Drehrichtung der Rührwelle (16) und/oder der Trockensubstanzanteil des Gärguts in Abhängigkeit von der Abweichung des Messwerts Aᵢₛₜ vom Sollwert Aₛₒₗₗ geregelt wird.

4. Verfahren nach einem der vorhergehenden Ansprüche, **dadurch gekennzeichnet, dass** der Bereich, innerhalb welchem die Drehzahl der Rührwelle (16) eingestellt wird, zwischen 0 und 10, vorzugsweise zwischen 0 und 1, besonders bevorzugt zwischen 0,2 bis 0,6 Umdrehungen pro Minute liegt.

5. Verfahren nach einem der vorhergehenden Ansprüche, **dadurch gekennzeichnet, dass** der Trockensubstanzanteil des Gärguts im Bereich zwischen 5 bis 99%, vorzugsweise zwischen 15 bis 40 % liegt.

6. Verfahren nach einem der vorhergehenden Ansprüche, **dadurch gekennzeichnet, dass** in nachfolgender Reihenfolge
- für den Fall, dass der in Schritt a) erhaltene Messwert A1ᵢₛₜ des Drehmoments bzw. der Leistung höher liegt als ein vorgegebener Maximalwert A1ₘₐₓ, die Drehzahl reduziert wird,
- das Drehmoment bzw. die Leistung bei reduzierter Drehzahl gemessen wird und
- für den Fall, dass der in Schritt II) erhaltene Messwert A1_{ist,red} immer noch höher liegt als A1ₘₐₓ,
• das Drehmoment bzw. die Leistung nochmals reduziert wird, worauf wahlweise die Messung des Drehmoments bzw. der Leistung bei nochmalig reduzierter Drehzahl und die daraufhin vorgenommene nochmalige Reduktion der Drehzahl mindestens einmal, vorzugsweise mehrmals wiederholt werden, und/oder
• die Drehrichtung der Rührwelle (16) geändert wird.

7. Verfahren nach Anspruch 6, **dadurch gekennzeichnet, dass** nachfolgend auf die Änderung der Drehrichtung der Rührwelle (16) nach einer Zeitdauer t die Drehrichtung wieder geändert wird.

8. Verfahren nach einem der vorhergehenden Ansprüche, **dadurch gekennzeichnet, dass** für den Fall, dass der in Schritt a) erhaltene Messwert A1ᵢₛₜ des Drehmoments bzw. der Leistung tiefer liegt als ein vorgegebener Minimalwert A1ₘᵢₙ, die Drehzahl reduziert wird und die reduzierte Drehzahl beibehalten wird, solange der Messwert A1ᵢₛₜ tiefer liegt als A1ₘᵢₙ.

9. Pfropfenstromfermenter (10) zur anaeroben Vergärung organischer Abfälle, wobei der Pfropfenstromfermenter einen horizontal ausgerichteten Fermentertank (12) und ein Rührwerk (14) umfasst, welches eine das Innere des Fermentertanks (12) durchquerende Rührwelle (16) und mehrere an der Rührwelle (16) angeordnete, radial abstehende Paddel (18) sowie einen Antrieb (20; 200) umfasst und dazu geeignet ist, das Gärgut im Fermentertank (12) zu bewegen, **dadurch gekennzeichnet, dass** der Pfropfenstromfermenter (10) zudem mindestens eine Messeinrichtung zur Bestimmung mindestens eines für den jeweiligen Betriebszustand des Pfropfenstromfermenters charakteristischen Parameters umfasst sowie Einstellmittel (26), die derart ausgestaltet sind, ausgehend vom erhaltenen Messwert des Drehmoments und/oder der Leistung des Antriebs bzw. der Abweichung des Messwerts von einem vorgegebenen Sollwert die Drehzahl der Rührwelle (16), die Drehrichtung der Rührwelle (16) und/oder den Trockensubstanzanteil des Gärguts einzustellen.

10. Pfropfenstromfermenter gemäss Anspruch 9, **dadurch gekennzeichnet, dass** die Einstellmittel (26) in Form oder als Teil einer Regelungseinheit (260) vorliegen, die derart ausgestaltet ist, die Drehzahl der Rührwelle (16), die Drehrichtung der Rührwelle (16) und/oder den Trockensubstanzanteil des Gärguts ausgehend vom erhaltenen Messwert bzw. der Abweichung des Messwerts vom Sollwert zu regeln.

11. Pfropfenstromfermenter gemäss Anspruch 9 oder 10, **dadurch gekennzeichnet, dass** es sich beim Antrieb (20) um einen Motor, insbesondere einen Asynchronmotor (200), handelt, dem ein Frequenzumwandler (22) zugeordnet ist.

## Claims

1. A process for optimizing the operation of a plug-flow fermenter (10) for the anaerobic fermentation of organic wastes, wherein the plug-flow fermenter comprises a horizontally oriented fermenter tank (12) and a stirrer (14), which stirrer comprises a stirrer shaft (16) which traverses the interior of the fermenter tank (12) in an axial manner and multiple paddles (18) which are arranged on the stirrer shaft and protrude radially and also a drive (20; 200), and the fermentation material is moved in the fermenter tank (12) by means of the stirrer (14), **characterized in that**
a) the torque and/or the power of the drive (20; 200) is measured,
b) the particular measurement value A1_{actual} obtained in a) is compared with a predefined nominal value A1ₙₒₘᵢₙₐₗ, and
c) depending on the deviation of the measurement value A1_{actual} from the nominal value A1ₙₒₘᵢₙₐₗ, the rotational speed of the stirrer shaft (16), the rotational direction of the stirrer shaft (16) and/or the dry-substance portion of the fermentation material is adjusted.

2. The process as claimed in claim 1, **characterized in that** the adjustment of the dry-substance portion of the fermentation material is done via the amount of wetting agent introduced into the fermenter tank (12) .

3. The process as claimed in claim 1 or 2, **characterized in that** the rotational speed of the stirrer shaft (16), the rotational direction of the stirrer shaft (16) and/or the dry-substance portion of the fermentation material is regulated in step c) depending on the deviation of the measurement value A_{actual} from the nominal value Aₙₒₘᵢₙₐₗ.

4. The process as claimed in any of the preceding claims, **characterized in that** the range within which the rotational speed of the stirrer shaft (16) is adjusted is between 0 and 10, preferably between 0 and 1, particularly preferably between 0.2 to 0.6 revolutions per minute.

5. The process as claimed in any of the preceding claims, **characterized in that** the dry-substance portion of the fermentation material is within the range between 5 to 99%, preferably between 15 to 40%.

6. The process as claimed in any of the preceding claims, **characterized in that** in the following order
- if the measurement value A1_{actual}, as obtained in step a), of the torque or the power is higher than a predefined maximum value A1ₘₐₓ, the rotational speed is reduced,
- the torque or the power is measured at reduced rotational speed and
- if the measurement value A1_{actual,red}, as obtained in step II), is still higher than A1ₘₐₓ,
• the torque or the power is further reduced, whereupon the measurement of the torque or the power at further reduced rotational speed and the subsequently performed further reduction of the rotational speed are repeated at least once, preferably multiple times, as desired and/or
• the rotational direction of the stirrer shaft (16) is changed.

7. The process as claimed in claim 6, **characterized in that**, subsequent to the change in the rotational direction of the stirrer shaft (16), the rotational direction is changed after a period t.

8. The process as claimed in any of the preceding claims, **characterized in that**, if the measurement value A1_{actual}, as obtained in step a), of the torque or the power is lower than a predefined minimum value A1ₘᵢₙ, the rotational speed is reduced and the reduced rotational speed is maintained so long as the measurement value A1_{actual} is lower than A1ₘᵢₙ.

9. A plug-flow fermenter (10) for the anaerobic fermentation of organic wastes, wherein the plug-flow fermenter comprises a horizontally oriented fermenter tank (12) and a stirrer (14), which stirrer comprises a stirrer shaft (16) which traverses the interior of the fermenter tank (12) and multiple paddles (18) which are arranged on the stirrer shaft (16) and protrude radially and also a drive (20; 200) and which stirrer is suited to moving the fermentation material in the fermenter tank (12), **characterized in that** the plug-flow fermenter (10) additionally comprises at least one measurement mechanism for the determination of at least one parameter characteristic of the particular operating state of the plug-flow fermenter as well as adjustment means (26) designed to adjust the rotational speed of the stirrer shaft (16), the rotational direction of the stirrer shaft (16) and/or the dry-substance portion of the fermentation material on the basis of the measurement value of the torque and/or the power of the drive (20; 200) obtained or the deviation of the measurement value from a predefined nominal value.

10. The plug-flow fermenter as claimed in claim 9, **characterized in that** the adjustment means (26) are present in the form of or as part of a regulation unit (260) designed to regulate the rotational speed of the stirrer shaft (16), the rotational direction of the stirrer shaft (16) and/or the dry-substance portion of the fermentation material on the basis of the measurement value obtained or the deviation of the measurement value from the nominal value.

11. The plug-flow fermenter as claimed in claim 9 or 10, **characterized in that** the drive (20) is a motor, more particularly an asynchronous motor (200), to which a frequency converter (22) has been assigned.

## Revendications

1. Procédé d'optimisation du fonctionnement d'un fermenteur à écoulement piston (10) pour la digestion anaérobie de déchets organiques, le fermenteur à écoulement piston comprenant une cuve de fermentation (12) orientée horizontalement et un agitateur (14), lequel agitateur (14) comprend un arbre d'agitateur (16) traversant axialement l'intérieur de la cuve de fermentation (12) et plusieurs pales (18) saillant radialement, disposées au niveau de l'arbre d'agitateur ainsi qu'un entraînement (20 ; 200), et le digestat étant mis en mouvement dans la cuve de fermentation (12) au moyen de l'agitateur (14), **caractérisé en ce que**
a) le couple et/ou la puissance de l'entraînement (20 ; 200) sont mesurés,
b) chaque valeur de mesure A1ᵢₛₜ obtenue en a) est comparée avec une valeur de consigne prédéfinie A1ₛₒₗₗ, et
c) en fonction de l'écart de la valeur de mesure A1ᵢₛₜ par rapport à la valeur de consigne A1ₛₒₗₗ, la vitesse de rotation de l'arbre d'agitateur (16), le sens de rotation de l'arbre d'agitateur (16) et/ou la proportion de substances sèches du digestat sont ajustés.

2. Procédé selon la revendication 1, **caractérisé en ce que** l'ajustement de la proportion de substances sèches du digestat s'effectue par le biais de la quantité d'agent humectant introduite dans la cuve de fermentation (12).

3. Procédé selon la revendication 1 ou 2, **caractérisé en ce qu'**à l'étape c), la vitesse de rotation de l'arbre d'agitateur (16), le sens de rotation de l'arbre d'agitateur (16) et/ou la proportion de substances sèches du digestat sont réglés en fonction de l'écart de la valeur de mesure Aᵢₛₜ par rapport à la valeur de consigne Aₛₒₗₗ.

4. Procédé selon l'une quelconque des revendications précédentes, **caractérisé en ce que** la plage à l'intérieur de laquelle la vitesse de rotation de l'arbre d'agitateur (16) est ajustée est comprise entre 0 et 10, de préférence entre 0 et 1, particulièrement préférablement entre 0,2 et 0,6 tr/min.

5. Procédé selon l'une quelconque des revendications précédentes, **caractérisé en ce que** la proportion de substances sèches du digestat est comprise dans une plage de 5 à 99 %, de préférence de 15 à 40 %.

6. Procédé selon l'une quelconque des revendications précédentes, **caractérisé en ce que** dans la séquence suivante
- au cas où la valeur de mesure A1ᵢₛₜ du couple ou de la puissance obtenue à l'étape a) est supérieure à une valeur maximale prédéfinie A1ₘₐₓ, la vitesse de rotation est réduite,
- le couple ou la puissance à vitesse de rotation réduite est mesuré(e) et
- au cas où la valeur de mesure A1_{ist,red} obtenue à l'étape II) est encore supérieure à A1ₘₐₓ,
• le couple ou la puissance est encore réduit(e), après quoi, au choix, on répète au moins une fois, de préférence plusieurs fois, la mesure du couple ou de la puissance à la vitesse de rotation encore une fois réduite et la réduction ensuite effectuée encore une fois de la vitesse de rotation et/ou
• le sens de rotation de l'arbre d'agitateur (16) est modifié.

7. Procédé selon la revendication 6, **caractérisé en ce que** suite à la modification du sens de rotation de l'arbre d'agitateur (16), après une durée t, le sens de rotation est à nouveau modifié.

8. Procédé selon l'une quelconque des revendications précédentes, **caractérisé en ce qu'**au cas où la valeur de mesure A1ᵢₛₜ du couple ou de la puissance obtenue à l'étape a) est inférieure à une valeur minimale prédéfinie A1ₘᵢₙ, la vitesse de rotation est réduite et la vitesse de rotation réduite est conservée tant que la valeur de mesure A1ᵢₛₜ est inférieure à A1ₘᵢₙ.

9. Fermenteur à écoulement piston (10) pour la digestion anaérobie de déchets organiques, le fermenteur à écoulement piston comprenant une cuve de fermentation (12) orientée horizontalement et un agitateur (14), lequel agitateur (14) comprend un arbre d'agitateur (16) traversant l'intérieur de la cuve de fermentation (12) et plusieurs pales (18) saillant radialement, disposées au niveau de l'arbre d'agitateur (16) ainsi qu'un entraînement (20 ; 200) et est prévu pour mettre en mouvement le digestat dans la cuve de fermentation (12), **caractérisé en ce que** le fermenteur à écoulement piston (10) comprend en outre au moins un dispositif de mesure pour déterminer au moins un paramètre caractéristique de l'état de fonctionnement respectif du fermenteur à écoulement piston ainsi que des moyens d'ajustement (26) qui sont configurés de manière à ajuster la vitesse de rotation de l'arbre d'agitateur (16), le sens de rotation de l'arbre d'agitateur (16) et/ou la proportion de substances sèches du digestat, à partir de la valeur de mesure obtenue du couple et/ou de la puissance de l'entraînement ou de l'écart de la valeur de mesure par rapport à une valeur de consigne prédéfinie.

10. Fermenteur à écoulement piston selon la revendication 9, **caractérisé en ce que** les moyens d'ajustement (26) se présentent sous la forme, ou en tant que partie, d'une unité de régulation (260) qui est configurée de manière à régler la vitesse de rotation de l'arbre d'agitateur (16), le sens de rotation de l'arbre d'agitateur (16) et/ou la proportion de substances sèches du digestat à partir de la valeur de mesure obtenue ou de l'écart de la valeur de mesure par rapport à la valeur de consigne.

11. Fermenteur à écoulement piston selon la revendication 9 ou 10, **caractérisé en ce que** l'entraînement (20) est un moteur, en particulier un moteur asynchrone (200) auquel est associé un convertisseur de fréquence (22).
